# EUROPEAN PATENT SPECIFICATION

(11) **EP 1 846 467 B1**
(45) Date of publication and mention of the grant of the patent: **21.09.2011**
(21) Application number: 06717828.5
(22) Date of filing: 09.01.2006
(51) Int. Cl.: C08G 18/10, C08G 18/42, C08G 18/76, A61L 24/04, C08G 18/30

(54) **DIISOCYANATE TERMINATED MACROMER AND FORMULATION THEREOF FOR USE AS AN INTERNAL ADHESIVE OR SEALANT**
DIISOCYANATTERMINIERTES MAKROMER UND FORMULIERUNG DARAUS ZUR VERWENDUNG ALS INNENHAFT- ODER DICHTUNGSMITTEL
MACROMERE A TERMINAISON DIISOCYANATE ET PREPARATION ASSOCIEE UTILISEE EN TANT QU'AGENT ADHESIF OU DE SCELLEMENT INTERNE

(30) Priority: 10.01.2005 US 32332
(43) Date of publication of application: 24.10.2007
(73) Proprietor: ETHICON, INC., Somerville, New Jersey 08876-0151 (US)
(72) Inventor: FITZ, Benjamin D., Brooklyn, New York 11225 (US)
(74) Representative: James, Anthony Christopher W.P.
(86) International application number: PCT/US2006/000674
(87) International publication number: WO 2006/076291

(56) References cited:
- WO-A-89/00589

## Description

### Field of the Invention

Described herein is a novel polyisocyanate macromer and the use thereof to form an internal adhesive or sealant for use in cardiovascular, peripheral-vascular, cardio-thoracic, gynecological, neuro- and general abdominal surgeries. More particularly, the macromer or a formulation thereof polymerizes in the human body to form an elastic gel that is biocompatible and that degrades into products that are non-toxic and biocompatible. Additionally, the degradation products are water soluble, allowing for the degradation products to be eliminated from the human body as waste products.

### Background of the Invention

Generally, the key requirements of a tissue adhesive are:
(1) In use, the adhesive must mimic the mechanical performance of the undamaged tissue;
(2) The adhesive should provide sufficient tack for "primary" fixation with the opportunity for manipulation and re-alignment prior to setting strongly;
(3) Any exothermic process involved in the curing of the adhesive should not damage the surrounding tissue;
(4) The adhesive must not elicit any toxic response by the surrounding healthy tissue and should facilitate the re-growth of new tissue where possible;
(5) The adhesive should not liberate harmful degradation products;
(6) The adhesive should degrade, and as it does so, it should be replaced by new tissue with minimal scarring; and
(7) Any biodegradation products should not accumulate in the body but should be eliminated naturally either by excretion or incorporation into the natural biochemical cycle.

### ["Polymeric Biomaterials", 2^{nd} Ed., Marcel Dekker Inc., (2002) pp. 716]

It is well known in the art that diisocyanate monomers may be used to form polymeric adhesives. However, many of the diisocyanate monomers that are commercially available are small molecule diisocyanate monomers that present toxicity and sensitization hazards and that polymerize to form products having toxic degradation products, for instance, aromatic amines. As such, commercially available small molecule diisocyanate monomers are unsuitable for human use as an internal adhesive or sealant.

Metabolically acceptable polyisocyanate monomers are described in USP 4,829,099. More specifically, this reference describes an aromatic benzoyl isocyanate terminated monomer, having glycolic acid residues and polyethyleneglycol residues, in formula "I, Preferred". This reference indicates that the resultant polymer will degrade ultimately to metabolically acceptable products, including p-aminobenzoic acid, polyethylene glycol and glycolic acid. Although the resultant polymer in principal could degrade into the aforementioned compounds, it is believed that only the glycolic acid residues would hydrolyse *in vivo,* resulting in a mixture of water-soluble and water insoluble fragments. The water-soluble fragments would be eliminated naturally by excretion from the body. However, the water insoluble fragments would not be eliminated naturally, resulting in the undesirable accumulation of the water insoluble fragments in the body.

Polyester-urethane-urea block copolymers prepared from commercially available small molecular diisocyanates, i.e. tolylene diisocyanate (TDI), diphenylmethane -4,4'-diisocyanate (MDI), and hexamethylene disisocyanate (HMDI), are described in USP 6,210,441. However, these copolymers would be unsuitable for use as a surgical adhesive or sealant, since the copolymers are already polymerized, i.e., already cured, and would not provide sufficient opportunity for manipulation and re-alignment. Moreover, such copolymers are not believed to mimic the mechanical performance of undamaged tissue.

Therefore, it is desirable to have a monomer based internal adhesive or sealant formulation that is capable of polymerizing *in vivo* to form an internal adhesive or sealant, in order to provide an opportunity for manipulation and re-alignment. Specifically, it is desirable that the adhesive or sealant formulation fill internal cavities and voids, penetrating and conforming to the interstices and pores of the tissue, prior to curing or setting.

Additionally, it is desirable to have a monomer based internal adhesive or sealant formulation that polymerizes *in vivo,* where the monomer, the formulation thereof, and the resultant polymer are biocompatible. The resultant polymer should also be biodegradable.

Finally, it is desirable that the degradation products of the resultant polymer be both biocompatible and water soluble, so that the degradation products are completely eliminated from the human body as waste products.

### Summary of the Invention

A novel macromer is described herein, comprising benzoyl isocyanate terminal moieties and at least two residues of a water-soluble polymer having a molecular weight ranging from 80 to 10,000 adjacent to the carbonyl group of the benzoyl isocyanate moieties, thereby forming at least two ester linkages in the macromer.

### Definitions

Unless defined otherwise, all technical and scientific terms used herein have the same meaning as is commonly understood by one of skill in the art to which this invention belongs. All patents and publications mentioned herein are incorporated by reference.

"Biocompatible" as used herein refers to a material that, once implanted, does not interfere significantly with wound healing and/or tissue regeneration, and does not cause any significant metabolic disturbance.

"Biodegradable" and "bioabsorbable" as used herein refer to a material that is broken down spontaneously and/or by the mammalian body into components which are consumed or eliminated in such a manner as not to interfere significantly with wound healing and/or tissue regeneration, and without causing any significant metabolic disturbance.

"Water-soluble polymer" as used herein refers to a polymer, which dissolve in water forming transparent solutions under ambient conditions (e.g. body temperature).

"Polyisocyanate" as used herein refers to a compound with two or more isocyanate groups.

"Urethane linkage" as used herein refers to a residue derived from a urethane moiety and having a carbonyl-containing functional group in which the carbonyl carbon is bound both to an ether oxygen and to an amine nitrogen: 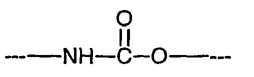 ["Organic Chemistry", J. McMurry, 2nd ed., Brooks/Cole Publishing Company, (1988), pp 1129]

"Urea linkage" as used herein refers to a residue derived from a moiety having a carbonyl-containing functional group in which the carbonyl carbon is bound to identical units of amine nitrogen: 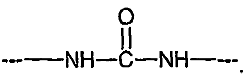 ["Nomenclature of Organic Chemistry", Pergamon Press, Oxford, (1979)]

### Detailed Description of the Invention

The present invention is defined in the claims.

As described above, a monomer based internal adhesive or sealant formulation that is capable of polymerizing *in vivo* to form an internal adhesive or sealant, should wet the tissue to which it is applied, penetrating and conforming to the interstices and pores of the tissue, prior to curing or setting. Additionally, the monomer, the formulation thereof, and the resultant polymer should be biocompatible.

The monomer and the formulation thereof described herein are suitable for internal applications, since neither the monomer, the formulation thereof nor the resultant polymer metabolizes In the human body to form toxic products.

Additionally, the monomer and the formulation thereof polymerize to form a biocompatible polymer upon contact with water or body fluids. The biocompatible polymer then degrades *in vivo* to form degradation products that are both biocompatible and water soluble, which are then eliminated from the human body as waste products.

The monomer and the formulation thereof have multiple medical applications, for example, as an internal surgical adhesive or sealant, a filler (e.g., dead space removal, reconstructive and cosmetic surgeries), as a matrix for tissue engineering (scaffolds), as a delivery matrix for cells, other biologics, bioactive agents and pharmaceutical or neutraceutical agents or adhesion prevention barriers. The monomer and the formulation thereof may be used in many types of surgery, including, but not limited to, cardiovascular, peripheral-vascular, cardio-thoracic, gynecological, neuro- and general abdominal surgery.

### Macromer

The monomer described herein is a biocompatible polyisocyanate macromer, terminating with benzoyl isocyanate groups and having the structural formula I: 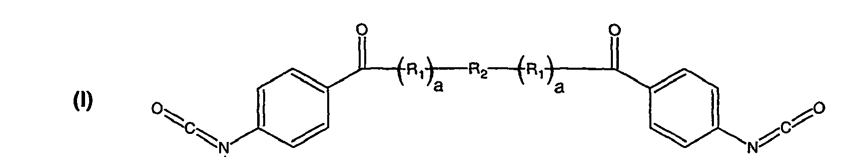 where R₁ is an organic residue containing a urethane linkage that is attached to R₂ when the value of "a" is one or more, and preferably one to five. The value of "a" in formula I may also be zero.

An example of R₁ when "a" is one or more is shown below: 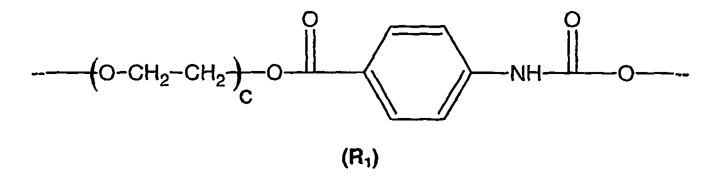 where the ethylene oxide portion of R₁ may be linear or branched, and c may range from 1 to 100, and preferably from 1 to 10.

The general structure of R₂ in formula I is the following: 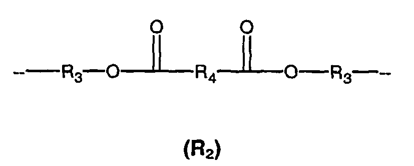 Where R2 in formula I has hydrolysable ester linkages that are biodegradable *in vivo.*

R3 may be residue of a water soluble polymer, including but not limited to a residue of a polyalkylene glycol such as polyethylene glycol, a polyalkylene oxide, polyvinylpyrolidone, poly(vinyl alcohol), poly(vinyl methyl ether), polyhydroxymethyl methacrylate, a polyacrylic acid polymer and copolymer, polyoxazoline, polyphosphazine, polyacrylamide, a polypeptide, or the water-soluble derivatives of any of the above, that is capable of forming ester linkages together with R4, and (i) ester linkages together with the carbonyl group of the benzoyl isocyanate moiety when "a" is zero or (ii) urethane linkages together with **R1** when "a" is one or more. Further, **R3** may be linear or branched. When **R3** is a polyethylene glycol residue, -(OCH₂CH₂) n⁻ , and "a" is one or more, n should be sufficiently large to render the degradation product **IV** (shown below) water soluble. For example, n may range from 2 to 250, preferably from 5 to 100, and more preferably is 5 to 25. The molecular weight of **R3** may range from 80 to 10,000, preferably 200 to 4000, and more preferably 200 to 1000. These residues of water- soluble polymer must be coupled into the macromer in the R3 position and are critical to the solubility of the degradation products, as will be discussed in more detail below.

**R4** may be an organic residue capable of having carboxylate end-groups. For example, **R4** may be a residue of, for example, diglycolic acid, malonic acid, succinic acid, glutaric acid, adipic acid, tartaric acid, and carboxylic acid terminated-polyalkyleneglycols. If **R4** is an aliphatic dicarboxylate: OC=O(CH₂)ₘC=OO, *m* may range from 1 to 10. The selection of *m* is based on two factors: biocompatibility and solubility of degradation products. If *m* is 0, the diacid hydrolytic degradation product of the macromer is too acidic, thus detrimental to biocompatibility of the composition. If *m* is too large, the diacid degradation product will no longer be water soluble.

Examples of **R2** includes but is not limited to a residue of a PEG-ester made from the polycondensation reaction of polyethylene glycol and a compound bearing multiple carboxylic groups, wherein the carboxylic group containing compounds include but are not limited to diglycolic acid, malonic acid, succinic acid, glutaric acid, adipic acid, tartaric acid, and carboxylic acid terminated-polyalkyleneglycols.

Examples of a PEG-ester version of **R₂** residue include but are not limited to:
a.) -(OCH2CH2)n-(OC=OCH2OCH2C=OO)-(CH2CH2O)n-
   where n is 20 for PEG of Mw 900 and the diacid is diglycolic acid
b.) -(OCH2CH2)n-(OC=OCH2CH2C=OO)-(CH2CH2O)n-
   where n is 20 for PEG of Mw 900 and the diacid is succinic acid
c.) -(OCH2CH2)n-(OC=OCH2CH2CH2C=OO)-(CH2CH2O)n-
   where n is 20 for PEG of Mw 900 and the diacid is glutaric acid
d.) -(OCH2CH2)n-(OC=OCH2CH2CH2CH2C=OO)-(CH2CH2O)n-
   where n is 20 for PEG of Mw 900 and the diacid is adipic acid

Other examples include branched R₂ residues:

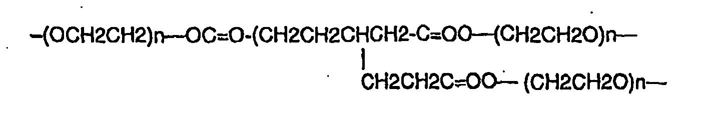

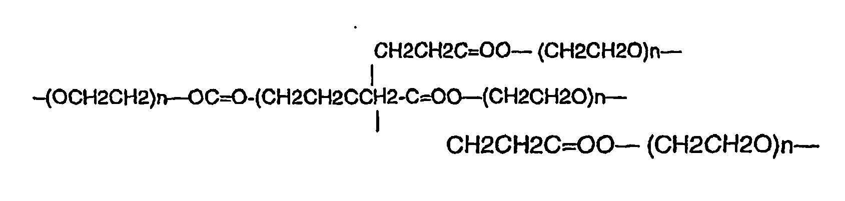

The molecular weight of the R₂ residue portion of the macromer may range from about 80 to 20,000g/mol.

Also disclosed herein is a polyisocyanate macromer represented by formula II: 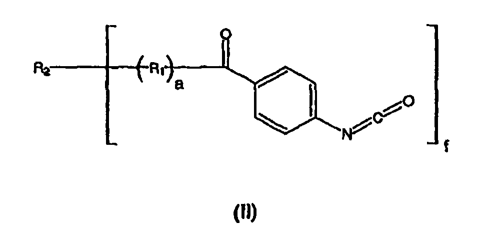 Wherein f represent the number of end-groups In the macromer.

When *f*=2, formula II represents a linear macromer, when *f* is three or more, formula II represents a branched macromer.

Examples of linear macromers include those shown In Formulae la and Ib.

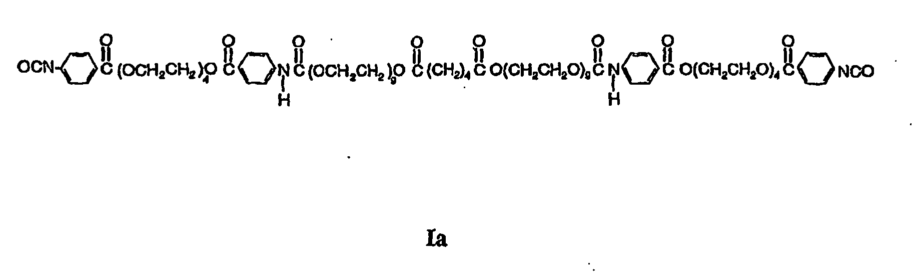

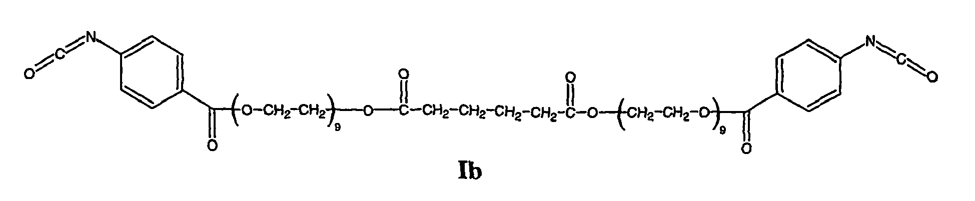

An example of a branched macromer is **IIa:**

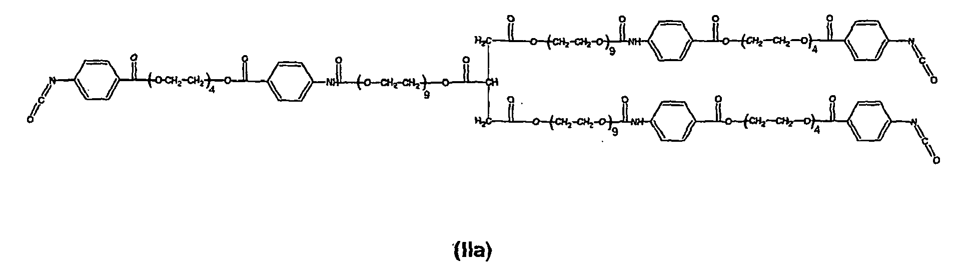

An alternative type of branched macromer is shown below as **III.** These are prepared by coupling an excess of linear isocyanate-terminated macromers of formula I with a multifunctional active hydrogen-terminated compound, such as a hydroxy-terminated compound, as shown here in **R6:**

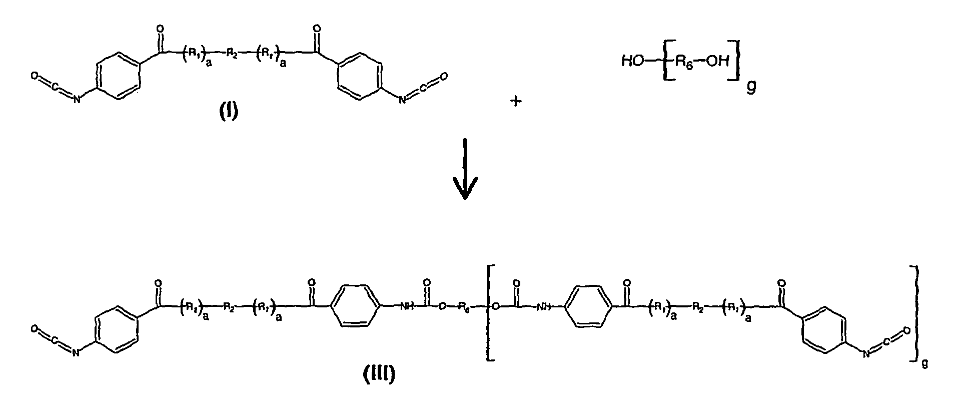

Wherein the polyol has g+1 hydroxyl end groups.

The molecular weight and degree of branching of the macromer are an important factors for determining biomechanical properties, such as elasticity, adhesive and cohesive strength, viscosity, absorption and water-uptake (swelling).

| **Property** | **Range** | **Preferred Range for Sealant** | **Preferred Range for Adhesive** |
|---|---|---|---|
| elasticity¹ | 10-2000% | 50-500% | 10-50% |
| adhesive strength² | burst pressure: >200mmHg | >200mmHg | lap shear tensile strength >1MPa |
| cohesive strength³ | 0.1-30MPa | 0.1-5 MPa | 5-25MPa |

| | | | |
|---|---|---|---|
| ²Adhesive strength quantifies the ability of the adhesive/sealant material to adhere to the biological tissue. It is measured by the fluid burst pressure test-ASTM 2392-04 - Burst pressure testing is performed by cutting a linear incision of 0.5 cm in a substrate (pericardium, dura or collagen) and placing the substrate in a test fixture. Sealant is applied to the incision and allowed to cure. Increasing pressure is applied to the transverse side of the substrate using a syringe pump filled with fluid. The maximum pressure is recorded when the sealant ruptures. ^{1,3}Cohesive strength refers to the intrinsic ability of adhesive/sealant material to withstand tensile forces. Cohesive strength and elasticity are measured by Elongation and Modulus - Tensile specimens of cured sealant are prepared by casting as a film. The samples are tested in tension at 1 inch/minute until failure. The maximum load and elongation at failure are recorded. | | | |

The range of the molecular weight of the macromer of formula III may be between about 500 to 20,000 g/mol, and preferably between about 500 and about 4000 g/mol.

### Macromer-Containing Formulation:

A medically acceptable formulation may comprise the polyisocyanate macromer, a solvent, a catalyst, a surfactant, a stabilizer or antioxidant, and a color additive.

Typically, the solvent is a hydrophilic solvent, including but not limited to dimethyl sulfoxide (DMSO), acetone, dimethoxy PEGs, glycerine, Tween 80, dimethylisosorbide, propylene carbonate, and 1-methyl-2-pyrrolidinone (NMP). Less hydrophillic solvents may also be considered, such as: ethyl lactate, triacetin, benzyl alcohol, benzylbenzoate, various ester solvents, such as: triethyl citrate, acetyltriethyl citrate, tri-n-butyl citrate, acetyltri-n-butyl citrate, ethyl acetate and the like. For example, the solvent may be used in an amount up to about 50 weight % based on the total weight of solvent and macromer.

The solvent plays several roles in the macromer formulation: (1) viscosity control, (2) control of bubble/foam formation and bubble escape, (3) to enhance tissue penetration, and (4) to provide improved tissue wetting. The viscosity of the formulation ranges from 10 to 100,000 cp, preferably from 500 to 50,000cp.

Surfactants may also be added to the formulation to control foaming: non-ionic surfactants such as Tween, Brij and siloxanes, as well as ionic surfactants, such as lecithin (phosphatidyl choline), sodium dodecyl sulfate, among others known in the arts.

Catalysts may also be added to the formulation for to increase reaction speed, such as triethylene diamine (DABCO), pyridine, ethyl-2-pyridyl acetate, and stannous octoate.

The color additive that may be utilized in the macromer formulation includes, but is not limited to, methylene blue, FD&C Blue #1 or #2, and conventional color additives that are used in absorbable medical devices such as sutures.

Antioxidants such as butylated hydroxyl toluene (BHT) may be present in the macromer formulation to improve shelf stability of the product.

### Adhesive System

One example of an adhesive system includes, but is not limited to, a system where the macromer and a solvent are stored separately until ready for use. For example, the macromer may be stored in one barrel of a double barrel syringe while the solvent is stored in the other barrel. Alternatively, the macromer and the solvent may be mixed by any conventionally means prior to use.

### Biocompatible Elastic Gel

The resultant polymer after the *in vivo* polymerization of the macromer is an elastic gel that is biodegradable, and the degradation products thereof should be both biocompatible and water soluble, so that the degradation products are completely eliminated from the human body as waste products.

Specifically, the macromer or formulation thereof polymerizes to form a biocompatible elastic gel upon contact with water or body fluids, via the following reaction scheme:
O=C=N-X-N=C=O (macromer) + H₂O produces HOOCHN-X-NHCOOH (carbamic acid), which spontaneously degrades under body conditions to H₂N-X-NH₂ + CO₂
Wherein X represent the structural component between the two terminal functional groups. X depends on the type of macromer such as formula I, II, or, III, as previously defined.

Then the H₂N-X-NH₂ reacts with an isocyanate group on another O=C=N-X-N=C=O (macromer) to form X- [NHCONH-X-]ₙ - (elastic gel)

The repeat unit of the gel is given in the following section, and may also be branched, depending on X.

### Degradation Products

The elastic gel formed from the macromer described herein is biodegradable and degrades by hydrolysis *in vivo* to form degradation products, including aromatic degradation products, that are both biocompatible and water soluble. In order to insure water solubility of any aromatic degradation product, the elastic gel is designed to cleave in such a way that the terminal groups on the aromatic degradation product are residues of water- soluble polymers. For example, after the macromer adhesive or sealant formulation polymerizes in the body, the elastic gel that results has the following repeat unit: 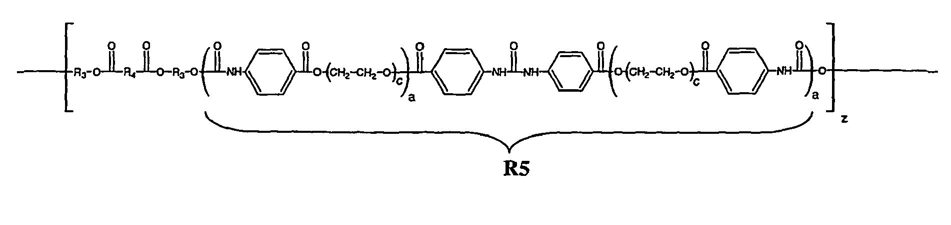 which is equivalent to

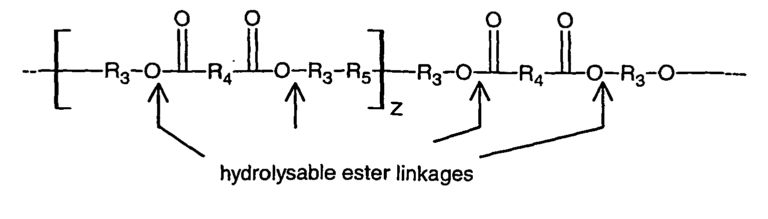

For example let us consider the biodegradation *in vivo* of a sealant made from PEG400-adipic acid PEG-ester converted into a urethane with PEG4-di benzoyl isocyanate, i.e., structure **Ia.** After implantation in the body, the elastic gel will initialy degrade hydrolytically into 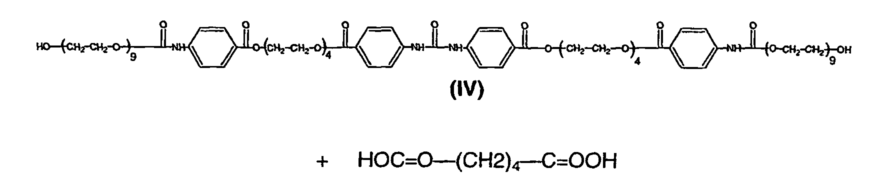 where all degradation products, including the aromatic degradation product, are essentially water soluble. In particular, the aromatic degradation product is solubilized by the presence of R3, a residue of a water-soluble polymer, as the terminal groups.

The biocompatible elastic gel that is formed comprises various hydrolysable linkages, including but not limited to, aliphatic and aromatic ester linkages, urethane linkages and urea linkages. The aliphatic ester linkages in the elastic gel have a higher tendency to degrade in vivo, than the other types of linkages, thereby leaving an initial aromatic degradation product IV containing the R5 aromatic fragment. While there are other linkages in the aromatic degradation product IV fragment that are susceptible to hydrolytic degradation (e.g., urethanes, and aromatic esters), for all practical purposes these do not degrade in vivo to any significant extent before the aromatic degradation product is excreted from the body. For example, the rapidly hydrolysable aliphatic ester linkages between **R3** and **R4** in the elastic gel degrade within 0-6 months; the more slowly hydrolysable aromatic ester linkages in the aromatic degradation product degrade within 4-24 months; the urethane linkages in the aromatic degradation product degrade within 4 to 24 months; and the very slowly hydrolysable urea linkages in the aromatic degradation product degrade within 24 month to infinity. During the timeframe from implantation of the macromer adhesive or sealant formulation to excretion of the aromatic degradation product **IV** from the body, degradation of the aromatic ester, urethane and urea linkages in the aromatic degradation product IV do not occur to any significant extent.

This composition has multiple medical applications. For example, as an internal surgical adhesive, the adhesive can bond tissue to tissue, tissue to medical device and medical device to medical device. As a sealant, the composition can be coated on a tissue, or on a medical device, or on the interface of a medical device with tissue to prevent leaks. The composition can be used to form films *in situ* that may have applications such as for the prevention of surgical adhesions. The composition can be used to form foams *in situ* that may have applications such as a filler (e.g. dead space removal, reconstructive and cosmetic surgeries), bulking agents, tissue engineering (e.g. scaffolds) materials and others where foams and sponges are useful. The composition can be formulated so that it is injectable and used to form gels *in situ* that are localized, and adherent to tissue, staying at the site where they are injected. These may have applications such as a delivery matrix for cells and other biologicals, bioactive agents and pharmaceutical or neutraceutical agents, and as embolization agents, and as means to localize contrasting agents. The composition may also be used to attach medical devices (e.g. meshes, clips and films) to tissues. This composition can be used internally in many types of surgery, including, but not limited to, cardiovascular, peripheral-vascular, cardio-thoracic, gynecological, neuro- and general abdominal surgery.

As a surgical sealant/adhesive, it can be used as an adjunct to primary wound closure devices, such as staples, sutures, to seal potential leaks of gasses, liquids, or solids. More specifically, the surgical adhesive/sealant may be applied to a tissue as a part of a surgical procedure, in various forms, for example: liquid, powder, film, sponge or foam, impregnated fabric, impregnated sponge or foam, or spray.

As a filler, the macromer or formulation thereof may be used as a facial, defect or void filler. For example, the formulation may be applied in the interstices of an internal void and allowed to polymerize therein, such that the polymer fills the internal cavities and voids, penetrating and conforming to the interstices and pores of the tissue. The formulation may be used after a broad number of procedures having potential risk of dead space formation, including, but not limited to, radical mastectomy (i.e. breast and regional lymph nodes removal for cancer treatment), breast reconstruction and augmentation procedure, reconstructive or cosmetic abdominoplasty and liposuction, face-lift, cesarean section and hysterectomy in obese patients, orthopedic procedures on thigh region, incisional hernia repair, lipoma excision, and traumatic lesions, i.e. closed trauma.

### Examples

While the following examples demonstrate certain embodiments of the invention, they are not to be interpreted as limiting the scope of the invention, but rather as contributing to a complete description of the invention.

### Example 1. Preparation of Polymers

### Comparative Prepolymer A1

A polyethylene glycol, Mw 900g/mol (50g, 0.056mol) was dried under vacuum at 120°C for four hours. Then the polymer was cooled to room temperature under nitrogen and glycolide (12.90g, 0.11mol) was added. Stannous octoate was added as a catalyst at 1 mol catalyst: 30,000 mol glycolide. The mixture was continuously stirred under nitrogen and heated to 150°C for 3 hours. Next the polymer was cooled to 70°C and paraphenylene diisocyanate (19.57g, 0.122mol) was added. This reaction continued under nitrogen with mixing for four hours. The theoretical structure of the resulting prepolymer is:

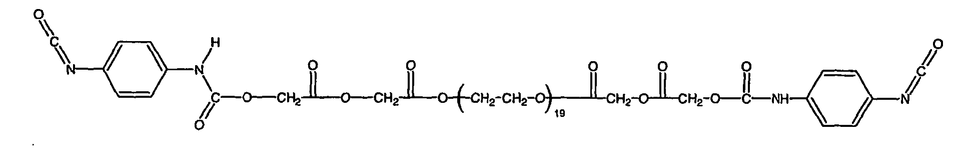

This polymer is a white waxy resin at room temperature.

### Prepolymer B1

A 10% solution of ethyl acetate was prepared with 1 mol of tetraethylene glycol, 2.75 mol of 4-nitro benzoyl chloride, and 6 equivalents of sodium carbonate. This reaction was carried out with magnetic stirring under nitrogen at room temperature and atmospheric pressure. The di-nitro intermediate: 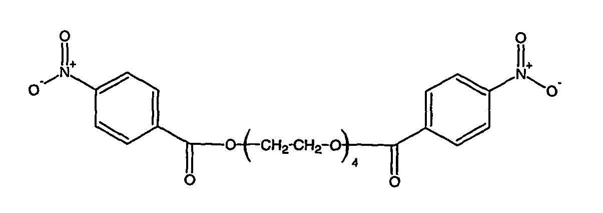 was next hydrogenated. To the ethyl acetate solution containing the dinitro intermediate palladium catalyst (10% Pd on carbon) was added at 5% w/w with vigorous stirring and a hydrogen sparge. This resulted in the di-amine intermediate:

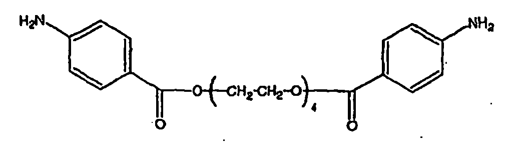

The diamine was purified by washing with aqueous sodium bicarbonate and brine, followed by drying over anhydrous magnesium sulfate. This diamine powder was then dried at 50°C under vacuum for 12 hours. The diamine was added to 1 equivalent of triphosgene and heated to 110C for three hours. Next the heat was increased to 130°C and vacuum was applied for 12 hours.
The resulting product is:

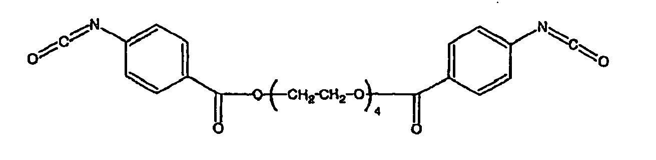

The structure was confirmed by NMR and %NCO titration. The purity was confirmed by performing HPLC on dibutylamine-blocked product. The product is an amber viscous liquid at room temperature.

### Prepolymer B2

Polyethyelene glycol, Mw 900g/mol (0.2 mol) was added to adipic acid (0.1 mol) with polymer bound para-toluene sulfonic acid, at 0.01 mol%. The mixture was heated to 160°C and water was condensed and distilled with the assistance of a nitrogen purge. Next a vacuum was applied for three hours. The resulting polyol: 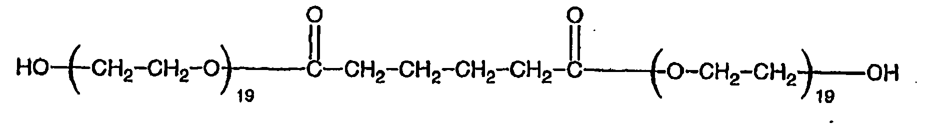 is a clear, colorless low viscosity liquid.

### Prepolymer B3

Two mol of prepolymer B1 are added to 1 mol of prepolymer B2 and were mixed and heated to 70°C for 8 hours under nitrogen. The resulting polymer: 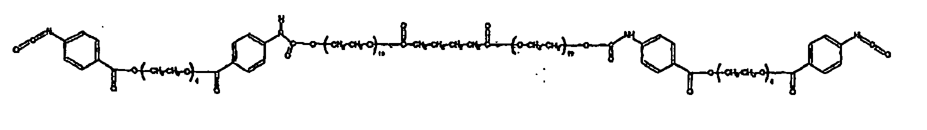 is a viscous amber liquid at room temperature.

### Example 2. Degradation Studies

The test polymer was cast onto glass and allowed to moisture cure under ambient humidity for several hours until a rubbery film was formed. The film was then subjected to the following accelerated hydrolysis conditions. The method consists of hydrolytically degrading a test specimen while maintaining a constant pH by titrating with a standard base and measuring the quantity of base used with time. This measurement and titration is automated by a pH stat instrument (718 STAT Titrator Complete, by MetroOhm, using Software TiNet 2.4). Samples are placed in a 70mL stirred, sealed, bath of deionized water held at 75°C+/-0.2°C, and at pH 7.27. Each sample bath is continuously monitored for pH changes (drops in pH) from the set point of 7.27. If any decrease is measured, sodium hydroxide solution is added to return to 7.27 (NaOH 0.05N). The hydrolysis continues until the titrating base is no longer needed to maintain the pH at 7.27. Any undissolved residue is collected, dried and weighed. The mass remaining is reported.

**Table 1. Degradation Studies**

| Mass remaining of degraded polymer. Degraded at 75°C, pH stat 7.27, for 10 days. | |
|---|---|
| **Composition** | **Wt. % remaining at end** |
| Comparative A1 | 30 |
| Inventive B3 | 0.5 |

Table 1 indicates that the water-solubility of the degradation product from the inventive composition B3 is far greater than that of the comparative composition of A1.

## Claims

1. A polyisocyanate macromer of the formula: 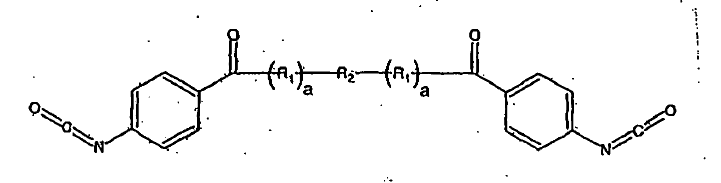 wherein "a" is one; and R₁ is 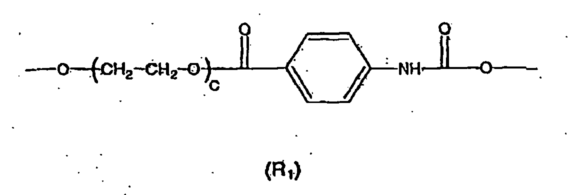 where the ethylene oxide portion of R₁ may be linear or branched, and c may range from 1 to 100;
R₂ is 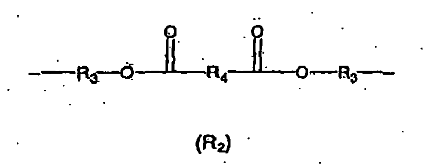 where R3 is a linear or branched residue of a water soluble polymer that is capable of forming ester linkages to R4, and urethane linkages to R1; and R4 is an organic residue capable of having carboxylate end-groups.

2. A polyisocyanate macromer of the formula:

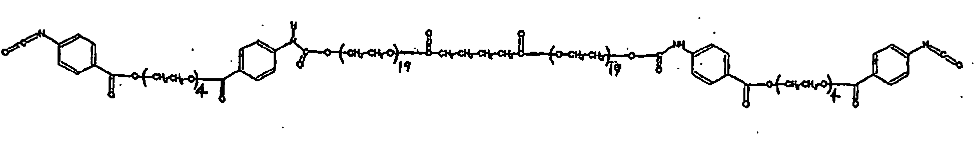

3. The macromer of claim 1, where R2 is selected from the group consisting of
-(OCH2CH2)n-(OC=OCH2OCH2C=OO)-(CH2H2O)n-,
-(OCH2CH2)n-(OC=O(CH2)mC=OO)-(CH2CH2O)n-, 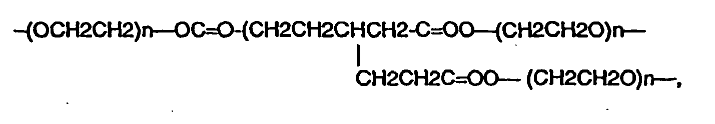 and 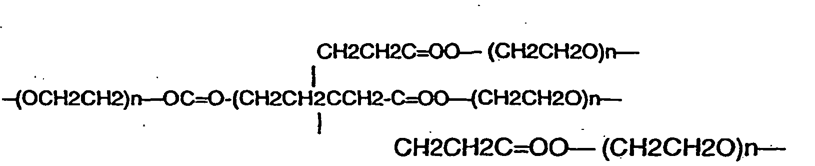 where n is from 2 to 250 and m is from 1 to 10.

4. The macromer of claim 1, where R3 is a residue of a compound selected from the group consisting of a polyalkylene glycol, a polyalkylene oxide, polyvinylpyrolidone, poly(vinyl alcohol), poly(vinyl methyl ether), polyhydroxymethyl methacrylate, a polyacrylic acid polymer and copolymer, polyoxazoline, polyphosphazine, polyacrylamide, a polypeptide, and water soluble derivative thereof; and R4 is a residue of a compound selected from the group consisting of diglycolic acid, malonic acid, succinic acid, glutaric acid, adipic acid, tartaric acid, and carboxylic acid terminated-polyalkyleneglycols.

5. A biocompatible polymer comprising the repeat unit: 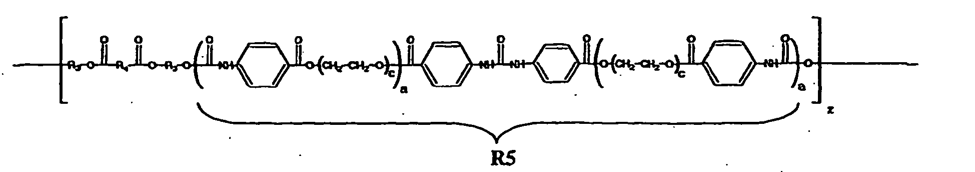 where R3 is a linear or branched residue of a water soluble polymer that is capable of forming ester linkages with R4, and urethane linkages with R5; and R4 is an organic residue capable of having carboxylate end-groups.

## Patentansprüche

1. Polyisocyanat-Makromer der Formel: 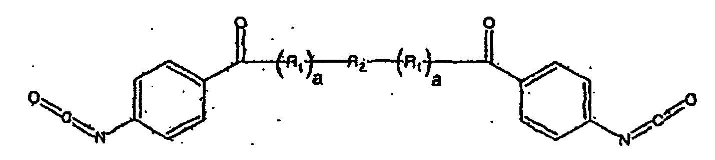 worin "a" für eins steht und R₁ für 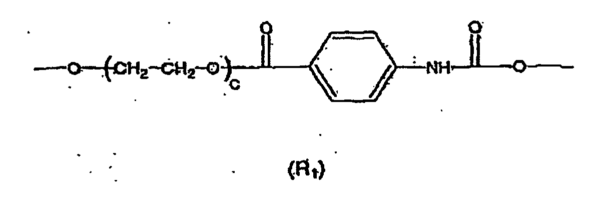 steht, wobei der Ethylenoxid-Teil von R₁ linear oder verzweigt sein kann, und c im Bereich von 1 bis 100 liegen kann;
R₂ für 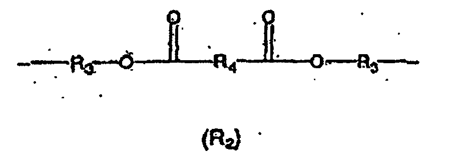 steht, wobei R₃ für einen linearen oder verzweigten Rest eines wasserlöslichen Polymers, das zur Ausbildung von Esterbindungen mit R₄ und Urethanbindungen mit R₁ befähigt ist, steht und R₄ für einen organischen Rest, der Carboxylat-Endgruppen aufweisen kann, steht.

2. Polyisocyanat-Makromer der Formel:

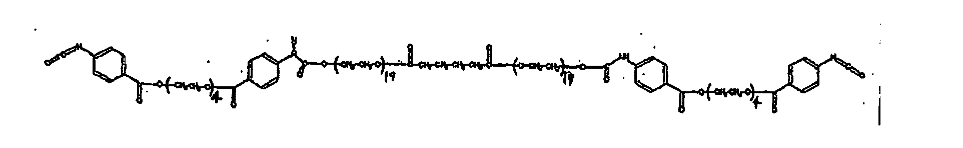

3. Makromer nach Anspruch 1, wobei R₂ aus der Gruppe bestehend aus
-(OCH2CH2)n-(OC=OCH2OCH2C=OO)-(CH2CH2O)n-,
-(OCH2CH2)n-(OC=O(CH2)mC=OO)-CH2CH2O)n-, 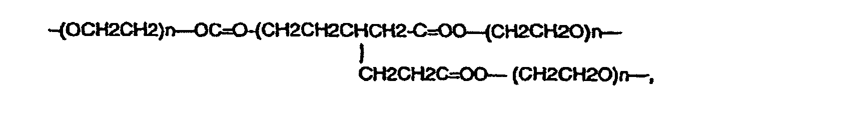 und 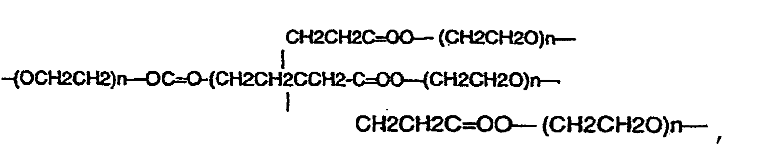 wobei n für 2 bis 250 steht und m für 1 bis 10 steht, ausgewählt ist.

4. Makromer nach Anspruch 1, wobei R₃ für einen Rest einer Verbindung aus der Gruppe bestehend aus einem Polyalkylenglykol, einem Polyalkylenoxid, Polyvinylpyrrolidon, Poly(vinylalkohol), Poly-(vinylmethylether), Polyhydroxymethylmethacrylat, einem Polyacrylsäurepolymer und -Copolymer, Polyoxazolin, Polyphosphazin, Polyacrylamid, einem Polypeptid und einem wasserlöslichen Derivat davon steht und R₄ für einen Rest einer Verbindung aus der Gruppe bestehend aus Diglykolsäure, Malonsäure, Bernsteinsäure, Glutarsäure, Adipinsäure, Weinsäure und carbonsäureterminierten Polyalkylenglykolen steht.

5. Biokompatibles Polymer, umfassend die Wiederholungseinheit: 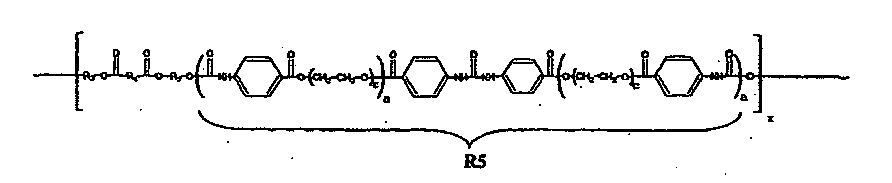 wobei R₃ für einen linearen oder verzweigten Rest eines wasserlöslichen Polymers, das zur Ausbildung von Esterbindungen mit R₄ und Urethanbindungen mit R₅ befähigt ist, steht und R₄ für einen organischen Rest, der Carboxylat-Endgruppen aufweisen kann, steht.

## Revendications

1. Macromère de polyisocyanate de formule : 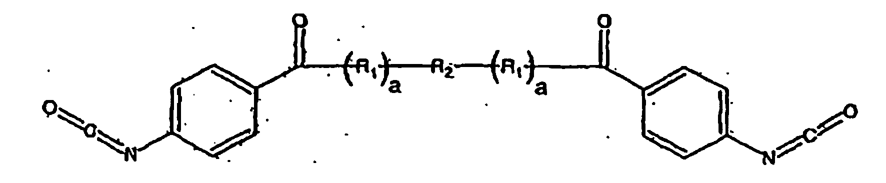 dans laquelle "a" est égal à un et R₁ représente 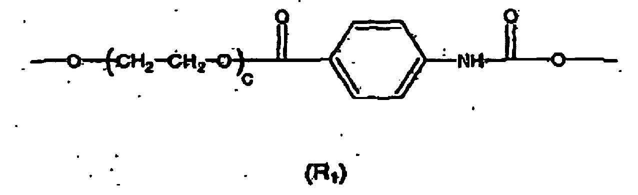 où la partie oxyde d'éthylène de R₁ peut être linéaire ou ramifiée, et c peut s'échelonner de 1 à 100 ;
R₂ représente 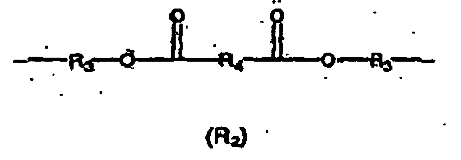 où R₃ représente un résidu linéaire ou ramifié d'un polymère soluble dans l'eau qui est capable de former des liaisons ester sur R₄ ; et des liaisons uréthane sur R₁ ; et R₄ représente un résidu organique capable d'avoir des groupes terminaux carboxylate.

2. Macromère de polyisocyanate de formule :

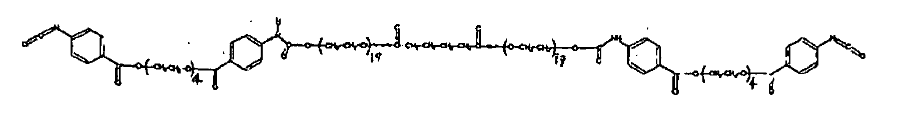

3. Macromère selon la revendication 1, dans lequel R₂ est choisi dans le groupe constitué par
-(OCH2CH2)n-(OC=OCH2OCH2C=OO)-(CH2CH2O)n-,
-(OCH2CH2)n-(OC-O(CH2)mC=OO) -(CH2CH2O)n-, 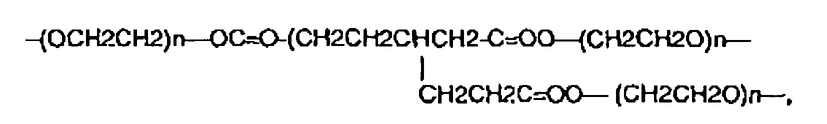 et 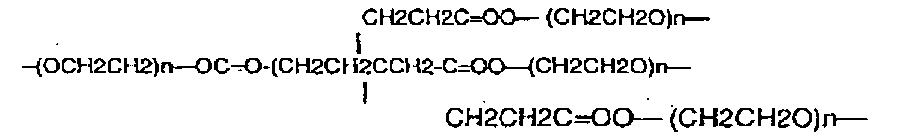 où n vaut de 2 à 250 et m vaut de 1 à 10.

4. Macromère selon la revendication 1, dans lequel R₃ est un résidu d'un composé choisi dans le groupe constitué par un polyalkylèneglycol, un poly(oxyde d'alkylène), une polyvinylpyrolidone, un poly(alcool vinylique), un poly(vinylméthyléther), un poly(méthacrylate d'hydroxyméthyle), un polymère et un copolymère de poly(acide acrylique), une polyoxazoline, une polyphosphazine, un polyacrylamide, un polypeptide, et un de leurs dérivés solubles dans l'eau ; et R₄ représente un résidu d'un composé choisi dans le groupe constitué par les polyalkylèneglycols à terminaison acide diglycolique, acide malonique, acide succinique, acide glutarique, acide adipique, acide tartrique et acide carboxylique.

5. Polymère biocompatible comprenant le motif récurrent : 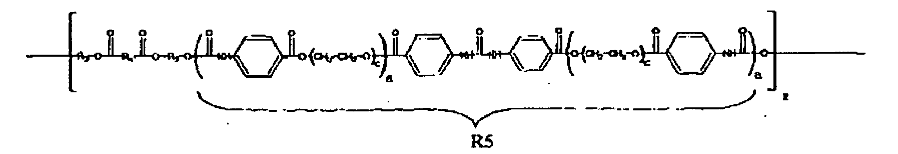 dans lequel R₃ est un résidu linéaire ou ramifié d'un polymère soluble dans l'eau qui est capable de former des liaisons ester avec R₄ et des liaisons uréthane avec R₅ ; et R₄ est un résidu organique capable d'avoir des groupes terminaux carboxylate.
